# EUROPEAN PATENT APPLICATION

(11) **EP 4 455 127 A1**
(43) Date of publication of application: **30.10.2024**
(21) Application number: 23170105.3
(22) Date of filing: 26.04.2023
(51) Int. Cl.: C07D 213/26, C07C 17/12, C07D 215/12, C07D 215/40, C07D 231/12, C07D 231/56, C07D 239/26, C07D 239/74, C07D 407/12, C07D 409/12

(54) **ARENE-CHLORINATION USING CHLORINATED WASTE**

(71) Applicant: ECOLE POLYTECHNIQUE FEDERALE DE LAUSANNE (EPFL), 1015 Lausanne (CH)
(72) Inventor: LIU, Mingyang, 1008 PRILLY (CH); DYSON, Paul, 1024 ECUBLENS VD (CH)
(74) Representative: Cohausz & Florack

(57) **Abstract**

Herein described is a method for chlorination of a substrate compound containing an arene moiety with 3 to 20 carbon atoms, wherein the substrate compound is reacted with an organic chlorine-donor in the presence of a catalyst, an oxidising agent, and a solvent. Also described is the use of a chlorine-containing solid or liquid organic compound, in particular a chlorinated organic waste compound, as chlorine-donor for the chlorination of a substrate compound containing an arene moiety with 3 to 20 carbon atoms.

## Description

The invention relates to a method for chlorination of a substrate compound containing an arene moiety and the use of a chlorine-containing solid or liquid organic compound, in particular an organic waste compound, as chlorine-donor for the chlorination of arenes.

### BACKGROUND

Chlorinated compounds are ubiquitous in industrialized societies as the physical and chemical properties of hydrocarbons are extensively modified and improved following chlorination. Thus, chlorine-containing hydrocarbons are widely used as plastics, rubbers, resins, solvents, and have applications as intermediates used in the synthesis of agrochemicals and pharmaceuticals or are even present in agrochemical and pharmaceutical products (see e.g. Naclerio, N. D.; Karsai, A.; Murray-Cooper, M.; Ozkan-Aydin, Y.; Aydin, E.; Goldman, D. I.; Hawkes, E. W., Sci. Robot. 2021, 6, eabe2922Jordan, A. et al., Chem. Rev. 2021, 121, 1582-1622; Bhutani, P.; Joshi, G.; Raja, N.; Bachhav, N.; Rajanna, P. K.; Bhutani, H.; Paul, A. T.; Kumar, R., J. Med. Chem. 2021, 64, 2339-2381).

Chlorine-containing hydrocarbons are widely used and may be persistent in the environment or decompose to release toxic compounds, which can accumulate in the food chain. Exposure to chlorinated waste is even associated with suppression of the immune system and cancer (see e.g. Z. Liew, P. Guo, Science 2022, 375, 720-721; A. L. Yee, J. A. Gilbert, Science 2016, 353, 348-349). However, methods to dispose of waste chlorine-containing hydrocarbons are currently inadequate.

Chlorine-containing hydrocarbon waste is one of the most problematic types of waste for several reasons. The common industrial approaches (landfill and incineration) lead to the formation of hazardous products including dioxins and corrosive gases, i.e. HCl and Clz. These problems are amplified by the large amounts of chlorine-containing hydrocarbon waste. Pyrolysis and hydrocracking are also unsuitable as they result in the same problems as industrial incineration. Disposal of chlorinated waste with low environmental impact is therefore both highly desirable and highly challenging (see e.g. Martin, A. J.; Mondelli, C.; Jaydev, S. D.; Pérez-Ramírez, J., Chem 2021, 7, 1487-1533; Ellis, L. D.; Rorrer, N. A.; Sullivan, K. P.; Otto, M.; McGeehan, J. E.; Román-Leshkov, Y.; Wierckx, N.; Beckham, G. T., Nat. Catal. 2021, 4, 539-556; C. Jehanno et al., Nature 2022, 603, 803-814).

Several methods have been reported in this respect.

For example, catalytic hydrocracking has been extensively investigated for upgrading a range of polymers. However, when applied to chlorinated polymers, catalyst deactivation is usually observed (see e.g. Chu, M.; Liu, Y.; Lou, X.; Zhang, Q.; Chen, J., ACS Catal. 2022, 12, 4659-4679; Lee, W.-T.; van Muyden, A.; Bobbink, F. D.; Mensi, M. D.; Carullo, J. R.; Dyson, P. J., Nat. Commun. 2022, 13, 4850; F. Zhang et al., Science 2020, 370, 437-441; R. J. Conk et al., Science 2022, 377, 1561-1566).

Further, a two-step process comprising dechlorination of the chlorinated waste followed by absorption of the resulting HCl and decomposition of the dechlorinated hydrocarbon compound has been reported. Although this process has a reduced environmental impact, it is expensive and largely unviable (see e.g. A. J. Martin et al., Chem 2021, 7, 1487-1533; Hou, Q.; Zhen, M.; Qian, H.; Nie, Y.; Bai, X.; Xia, T.; Laiq Ur Rehman, M.; Li, Q.; Ju, M., Cell Rep. Phys. Sci. 2021, 2, 100514).

Recently an electrochemical dechlorination method was reported for polyvinylchloride (PVC), but only partial dechlorination (< 20% conversion of the Cl groups) was achieved and the method might be difficult to upscale (see D. E. Fagnani et al., Nat. Chem. 2022, doi.org/10.1038/s41557-022-01078-w). Other reported dechlorination methods for chlorinated hydrocarbon compounds suffer from low reaction rates and low Faradaic efficiencies (typically < 50%; see e.g. G. Gan et al., ACS Catal. 2021, 11, 14284-14292; G. Gan et al., ACS Nano 2020, 14, 9929-9937; C. Choi et al., Nat. Nanotechnol. 2022).

In addition to chlorinated hydrocarbon waste such as PVC tubes, waste chlorinated solvents are problematic with incineration being the conventional method used to dispose of them as bioremediation is slow (see e.g. A. Alberini, J. Bartholomew, Contemp. Econ. Policy 1999, 17, 309-320; D. Pekelney, J. Hazard. Mater. 1990, 23, 293-315).

It follows from the prior art elucidated above that there is no sustainable, economically viable, and scalable method to eliminate different chlorinated hydrocarbon wastes. Thus, it is an object of the present invention to provide a method to eliminate chlorine waste without the formation of toxic by-products. Ideally, the hydrocarbon components are eliminated simultaneously. The method is preferably economically viable and/or scalable.

Other and further objects, features and advantages of the present invention will become apparent more fully from the following description.

### SUMMARY OF THE INVENTION

Some or all of these objects are achieved with the present invention by the method according to claim 1 and the use according to claim 15.

It was surprisingly found that in the method according to the invention chlorinated compounds, such as chlorinated solid and/or liquid waste streams, could be efficiently employed as chlorine donors in the chlorination of arene compounds. Thus, the present invention provides an efficient route to safely dispose of hazardous chlorinated waste by its dechlorination and conversion to CO₂, CO and H₂O, while the resulting chlorine compounds can be used as convenient chlorine donors for the chlorination of arene compounds to aryl chlorides. The present invention overcomes the limitations of the prior art in terms of environmentally benign disposal of chlorinated hydrocarbon waste and simultaneously affords high-value aryl chlorides in good yields without the production of hazardous by-products.

A range of aromatic and heteroaromatic compounds with arene moieties that have 3 to 20 carbon atoms are suitable substrates for the chlorination reaction, for example those based on pyridine or pyrimidine moieties. Additionally, substrates with electron-donating or -withdrawing substituents can also be chlorinated.

The arene moiety with 3 to 20 carbon atoms in the substrate compound preferably consists of a single aromatic or heteroaromatic ring or a fused aromatic or heteroaromatic ring system that may be substituted with non-aromatic substituents or with aromatic substituents that are not conjugated with the ring or the ring system. The amount of carbon atoms preferably refers to the amount of carbon atoms within the ring or the ring system and excludes the carbon atoms of optional non-aromatic substituents and the aromatic substituents that are not conjugated with the ring or the ring system. For example, in 2-(p-methoxyphenyl)pyridine, there are two arene moieties - an aromatic p-methoxyphenyl moiety having six carbon atoms connected to a heteroaromatic pyridine moiety having five carbon atoms. In 7,8-benzoquinoline, there is only one aromatic moiety having thirteen carbon atoms. The substrate compound may also consist of the aromatic moiety, as for example in 7,8-benzoquinoline.

### DETAILED DESCRIPTION

The invention provides for a method for the chlorination of a substrate compound containing an arene moiety with 3 to 20 carbon atoms in the presence of a catalyst, an oxidising agent, and a solvent, using an organic chlorine-donor.

Further advantageous embodiments of the invention are specified in the dependent claims and are elucidated in detail herein below.

Various organic chlorine-containing compounds can be used as organic chlorine-donors in the method of the present invention. According to an embodiment of the invention, the chlorine-donor contains a chlorinated hydrocarbon compound. The chlorine-donor can also consist of a chlorinated hydrocarbon compound. Chlorinated hydrocarbon compounds can in particular be relatively easily handled and can be less expensive than specialised chlorination reagents such as chloramines.

The chlorinated hydrocarbon compound can be solid or liquid at 25 °C. This simplifies the handling of the compounds and of the reaction mixture compared to gaseous chlorination reagents.

According to a preferred embodiment, the chlorinated hydrocarbon compound contains a chlorine atom connected to an aliphatic moiety. Preferably, the chlorinated hydrocarbon compound contains a tertiary alkyl chloride moiety, a secondary alkyl chloride moiety, and/or a primary alkyl chloride moiety. More preferably, the chlorinated hydrocarbon compound contains a secondary alkyl chloride moiety, and/or a primary alkyl chloride moiety. Most preferably, the chlorinated hydrocarbon compound contains a primary alkyl chloride moiety.

According to an embodiment, the chlorine-donor is selected from the group consisting of polyvinyl chloride, polyvinylidene chloride, polyepichlorohydrin, neoprene, poly(epichlorohydrin-co-ethylene oxide), poly(epichlorohydrin-co-CO₂), dichloromethane, chloroform, 1,2-dichloroethane, 1,1-dichloroethane, 1,1,2-trichloroethane, 1,2-dichloroethene, perchloroethene, 1,6-dichlorohexane, 2-chlorohexane, 1,4-dichlorobutene, chlorocyclohexane, (3-chlorobutyl)benzene, benzyl chloride, epichlorohydrin, epichlorohydrin carbonate, and mixtures thereof. Preferably, the chlorine-donor is selected from the group consisting of polyvinyl chloride, polyvinylidene chloride, polyepichlorohydrin, neoprene, poly(epichlorohydrin-co-ethylene oxide), poly(epichlorohydrin-co-CO₂), dichloromethane, 1,2-dichloroethane, 1,1-dichloroethane, 1,2-dichloroethene, 1,6-dichlorohexane, 2-chlorohexane, 1,4-dichlorobutene, (3-chlorobutyl)benzene, benzyl chloride, epichlorohydrin, epichlorohydrin carbonate, and mixtures thereof. In a particularly preferred embodiment, chlorine-donors are selected from the group consisting of polyvinyl chloride, polyvinylidene chloride, dichloromethane, 1,2-dichloroethane, benzyl chloride, and mixtures thereof. Most preferred is the use of polyvinyl chloride as chlorine-donor.

Advantageously, the chlorine-donor can be employed in the form of chlorinated solvent waste or post-consumer plastic waste, such as polyvinyl chloride pipes.

The chlorine-donor is advantageously employed in an amount of 1.0 to 5.0 equivalents, more preferably 1.0 to 3.0 equivalents, even more preferably 1.0 to 2.0 equivalents, most preferably 1.5 equivalents, based on the amount of substrate compound. The use of less than 1.0 equivalents could result in incomplete conversion, while the use of excessive amounts of chlorine-donor is disadvantageous from an economic perspective.

In the method according to the invention, a substrate compound containing an arene moiety is reacted with an organic chlorine-donor in the presence of a catalyst, an oxidising agent, and a solvent. Advantageously, the catalyst can be a heterogeneous or homogeneous, preferably a homogeneous, catalyst.

In a preferred embodiment of the method according to the invention, the catalyst contains a metal selected from the group consisting of copper, nickel, cobalt, palladium, and mixtures thereof. More preferably the metal is copper and/or palladium, even more preferably a palladium compound and/or a copper salt. According to an embodiment, the catalyst contains palladium, in particular a palladium compound. Preferred palladium compounds are PdO, Pd(PPh₃)₄, PdClz, and mixtures thereof. According to another embodiment, the catalyst contains copper, in particular a copper salt. Preferred copper salts are selected from the group consisting of CuClz, CuBr₂, CuI₂, Cu(OAc)₂, Cu(OTf)₂, CuSO₄, Cu(acac)₂, CuPF₆(CH₃CN)₂, CuO, and/or Cu(NO₃)₂, and mixtures thereof, more preferably selected from the group consisting of Cu(OAc)z, Cu(OTf)₂, CuSO₄, Cu(acac)₂, CuPF₆(CH₃CN)₂, CuO, and/or Cu(NO₃)₂, and mixtures thereof.

Thus according to an embodiment, the catalyst contains a palladium compound and/or a copper salt selected from the group consisting of PdO, CuClz, CuBr₂, CuI₂, Cu(OAc)z, Cu(Otf)₂, CuSO₄, Cu(acac)₂, CuPF₆(CH₃CN)₂, CuO, Cu(NO₃)₂, and mixtures thereof, preferably from the group consisting of PdO, Cu(OAc)₂, Cu(OTf)₂, CuSO₄, Cu(acac)₂, CuPF₆(CH₃CN)₂, CuO, Cu(NO₃)₂, and mixtures thereof. Most preferably, the substrate compound is reacted with the chlorine donor in the presence of Cu(NO₃)₂. These catalysts improve the yield of aryl chlorides significantly compared to the examples without catalyst or employing other catalysts.

The copper salts reported herein encompass the water-free form as well as the known hydrates of these salts.

The catalyst is advantageously present in the method according to the invention in an amount of 1 to 50 mol%, more preferably 5 to 40 mol%, even more preferably 10 to 30 mol%, most preferably 15 to 25 mol%, based on the amount of substrate compound. The use of less than 1 mol% catalyst may result in incomplete conversion and low reaction rates, while the use of excess amounts of catalyst higher than 50 mol% is costly and thus disadvantageous from an economic perspective.

According to a preferred embodiment of the method according to the invention, the catalyst is used in combination with a second catalyst, preferably a second metal catalyst. The second catalyst can be a heterogeneous or homogeneous catalyst, preferably a heterogeneous catalyst. The second catalyst is preferably a second metal catalyst selected from the group consisting of a palladium catalyst, a platinum catalyst, an iron catalyst, a nickel catalyst such as NiO, and mixtures thereof. More preferably, the second catalyst is a palladium catalyst. Even more preferably, the second catalyst is PdO, 10%Pd/C, Pd(OH)₂/C, Pd(OAC)₂, Pd(TFA)₂, Pd(dba)₂, Pd(acac)₂, Pd(NO₃)₂·2H₂O, and/or PdSO₄. Most preferably, the second catalyst is PdO. The use of PdO as second catalyst improves the yield of aryl chlorides compared to without a second catalyst or with different second catalysts.

If a second catalyst is used, it is advantageously present in an amount of 0.01 to 50 mol%, more preferably 0.1 to 30 mol%, even more preferably 1 to 15 mol%, most preferably 2.5 to 7.5 mol% based on the amount of substrate compound. The use of less than 0.01 mol% of the second catalyst may result in incomplete conversion, while the use of excess amounts of the second catalyst higher than 50 mol% is costly and thus disadvantageous from an economic perspective.

In the method according to the invention, the substrate compound is reacted with the chlorine donor in the presence of an oxidising agent. In a preferred embodiment, the oxidising agent is selected from the group consisting of oxygen, air, nitrate, ozone, hydrogen peroxide, inorganic peroxides, chlorine, nitric acid, permanganate, dichromate, chlorate, sulfuric acid, osmium tetroxide, sodium perborate, nitrogen oxide gases, sodium bismuthate, ceric ammonium nitrate, ceric sulfate, lead dioxide, and mixtures thereof. While other oxidising agents may be suitable as well, the ones mentioned here have the advantage of being readily available on an industrial scale.

In a preferred embodiment, the oxidising agent is a combination of a gaseous and a non-gaseous oxidising agent. For example, oxygen or air can be used in combination with hydrogen peroxide, permanganate or a nitrate salt. According to a preferred embodiment, the oxidising agent is a combination of oxygen and a nitrate salt, most preferably a combination of oxygen and sodium nitrate. In this way, a high yields of the aryl chloride products may be obtained.

In a preferred embodiment of the method according to the invention, a gaseous oxidising agent is employed, preferably oxygen.

The non-gaseous oxidising agent, preferably sodium nitrate, is advantageously employed in an amount of 1 to 100 mol%, preferably 5 to 80 mol%, more preferably 10 to 50 mol%, most preferably 20 mol% based on the amount of substrate compound. The use of lower amounts of the non-gaseous oxidising agent may result in incomplete conversion, while the use of excess amounts of non-gaseous oxidising agent higher than 100 mol% is costly and thus disadvantageous from an economic perspective.

The gaseous oxidising agent, preferably oxygen, is advantageously employed at a pressure of 1 to 100 bar, preferably at 1 to 50 bar, more preferably at 1 to 10 bar, most preferably at 3 bar. Lower pressures may result in incomplete conversion, while pressures exceeding 100 bar necessitate special equipment and are unsuitable from an industrial perspective.

In the method according to the invention, the substrate compound is reacted in the presence of a solvent. According to a preferred embodiment, the solvent is a proton-containing non-aromatic solvent, preferably a dipolar aprotic solvent, preferably dimethyl sulfoxide. The solvent preferably contains water in an amount of 0.001 to 1 wt.%, more preferably 0.05 to 1 wt.%, most preferably 0.05 to 0.1 wt.% based on the amount of solvent.

In the method according to the invention, the substrate compound contains an arene moiety with 3 to 20 carbon atoms. The substrate compound may contain more than one arene moiety. According to an embodiment, the substrate compound contains two arene moieties.

The substrate compound may contain one or more heteroatoms. According to an embodiment, the substrate compound contains 1 to 20 heteroatoms, preferably 1 to 10 heteroatoms. Heteroatoms may be incorporated into the arene moiety or into another part of the substrate compound.

In an embodiment, the arene moiety of the substrate compound contains 1 to 8, preferably 1 to 3, heteroatoms selected from the group containing nitrogen, oxygen, sulfur, and phosphorus, preferably nitrogen.

The chlorination preferably takes place on the arene moiety. If the substrate compound contains more than one arene moiety, chlorination may take place on each arene moiety. If the substrate compound contains more than one arene moiety and one of the arene moieties contains at least one heteroatom, in particular a nitrogen atom, chlorination preferably takes place on the arene moiety that does not contain a heteroatom, in particular a nitrogen atom.

In a further embodiment, the arene moiety of the substrate compound contains additional functional groups containing heteroatoms, in particular amide groups. The additional functional groups containing heteroatoms on the substrate compound may aid in directing the site at which chlorination takes place.

The method according to the invention can be conducted at various temperatures. Advantageously, the substrate compound is reacted with the chlorine donor at a temperature from 10 to 200 °C, preferably from 100 to 180 °C, more preferably from 130 to 150 °C. At these temperatures, the reaction proceeds sufficiently fast while the compounds are not degraded.

In a preferred embodiment of the method according to the invention, no use of electrodes and/or current is required for the reaction to proceed.

The invention also relates to the use of a chlorine-containing solid or liquid organic compound as chlorine-donor for the chlorination of a substrate compound containing an arene moiety with 3 to 20 carbon atoms.

According to an embodiment of the use according to the invention, the chlorine-containing solid or liquid organic compound is a chlorinated organic waste compound. Preferably, the chlorine-containing solid or liquid organic compound is selected from solid polymer waste such as post-consumer waste plastic and rubber, liquid laboratory waste such as chlorinated solvents, and mixtures thereof.

According to an embodiment of the use according to the invention, the chlorination is conducted in the presence of a catalyst, an oxidising agent, and a solvent. Preferably, the details regarding the catalyst, the oxidising agent, and the solvent as described for the method according to the invention also apply to the use according to the invention.

In the following, the invention is further described by way of examples that are in no way meant to be limiting.

### EXAMPLES

### Materials

Inorganic salts including Cu salts, Pd salts and nitrates, solvents, alkyl chlorides and substrate compounds containing an arene moiety (if not specified) were purchased from Sigma-Aldrich and used directly unless specified otherwise. Pure polymers including polyvinylidene chloride (PVDC) M_{w}≈50'000/10'000/250'000, polychloroprene (chloroprene rubber, CR), polyepichlorohydrin (PECH) M_{w}≈700'000, poly(epichlorohydrin-co-ethylene oxide) (PECHEO, epichlorohydrin 64-69 wt.%) M_{w}≈30'000 and poly(epichlorohydrin-co-CO₂) (PECHC) M_{w}≈20'000 were purchased from Sigma-Aldrich. Compressed air, N₂ and O₂ were purchased from Carbagas. Polyvinylchloride (PVC) or neoprene-based water pipe and electrical conduit were obtained from Semadeni AG, electric wire was obtained from Helukabel, and vacuum tube was obtained from maagtechnic AG. PVDC blisters (Ibuprofen N Zentiva) was purchased from SUN STORE Pharmacies in Lausanne. The equivalents of the chlorine-donor were calculated based on the content of chlorine in the material used, which for the post-consumer waste was determined by Schöniger combustion according to the method described in Schöniger, W., Mikrochim. Acta 1955, 43, 123-129, wherein ion chromatography was used for the determination of the chlorine concentration in the absorption solution as described in Section 2.3.2 of Ma, W., Hoffmann, G., Schirmer, M., Chen, G., Rotter, V. S., J. Hazard. Mater. 2010, 178, 489-498.

General reaction protocol for the chlorination of benzoquinoline as the substrate compound comprising an arene moiety using PVC water pipe as the chlorine-donor 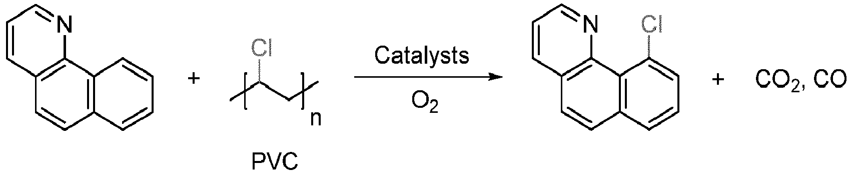 7,8-Benzoquinoline (0.25 mmol), PVC water pipe (1.5 equiv., 0.375 mmol), Cu(NO₃)₂ (20 mol%, 0.05 mmol), PdO (5 mol%, 0.0125 mmol), NaNO₃ (20 mol%, 0.05 mmol), biphenyl (0.2 mmol, internal standard) and DMSO (2 mL) were added into a glass insert vial of an autoclave and charged with Oz (3 bar). The reactor was heated to 140 °C for 15 h. After cooling to room temperature, the gas products were collected with a Tedlar^{®} gas sampling bag (Sigma-Aldrich) for analysis by gas chromatography. Ethyl acetate (3×3 mL) and an aqueous saturated brine solution (5 mL) were added to the reaction mixture to extract the organic products. The combined organic phase was used for analysis. Further purification was achieved using silica gel chromatography when required.

### Analytical Methods

Qualitative and quantitative analysis of gas phase products was performed by gas chromatography (GC) using an Agilent 7890B instrument equipped with a hydrogen flame-ionization detector (FID) and a thermal conductivity detector (TCD). The GC yield was determined based on standard gas mixtures and integrated peak areas using an external standard method. Qualitative and quantitative analysis of crude liquid products was performed using an Agilent 7000C GCMS equipped with a hydrogen FID, electron ionization mass detector (EI-MS) and HP-5 nonpolar column. The GC yield was determined based on internal standard curves and integrated peak areas.

Determination of the molecular weight and polydispersity of the polymers was performed using gel permeation chromatography (GPC, Agilent 390-MDS), equipped with a PL1513-5500 column (Agilent), refractive index detector (RI), and dual-angle light scattering detector. THF was used as eluent. For the measurement after reaction, to the DMSO solution (2 mL) brine (10 mL) was added and the organic components extracted with THF (3×5 mL). The THF extract was used for GPC.

¹H and ¹³C NMR spectra were recorded on a Bruker Avance III HD 400 instrument equipped with a 5 mm BBFO probe. DMSO-d₆ or CDCl₃ were used as solvent. DMSO-d₆ was used as the reaction solvent for the reaction that was monitored by ¹H NMR spectroscopy. After centrifuging to remove the PdO catalyst, the crude reaction mixture (0.2 mL) was diluted with DMSO-d₆ (0.4 mL) and directly used for ¹H NMR experiment.

According to the general reaction protocol described above, several syntheses as compiled in Table 1 were performed using different chlorine donors, and the yields as reported in Table 1 were obtained in these syntheses.

**Table 1: Syntheses performed using different chlorine donors and respective yields**

| Exp. No. | Chlorine donor | Equivalents^{a} | Yield^{b} |
|---|---|---|---|
| 1 | PVC water pipe | 1.5 | 99 |
| 2 | PVC electrical conduit | 1.5 | 80 |
| 3 | PVC electric wire | 1.5 | 96 |
| 4 | PVDC raw powder | 1.5 | 99 |
| 5 | PVDC blisters | 1.5 | 99 |
| 6 | PECH | 1.5 | 99 |
| 7 | Neoprene raw chunks | 1.5 | 80 |
| 8 | Neoprene vacuum tube | 1.5 | 82 |
| 9 | PECHEO | 1.5 | 99 |
| 10 | PECHC | 1.5 | 99 |
| 11 | Dichloromethane | 3 | 82 |
| 12 | Chloroform | 3 | 12 |
| 13 | 1,2-Dichloroethane | 3 | 83 |
| 14 | 1,1-Dichloroethane | 3 | 90 |
| 15 | 1,2,3-Trichloroethane | 3 | 40 |
| 16 | 1,2-Dichloroethene | 3 | 87 |
| 17 | Perchloroethene | 3 | 42 |
| 18 | 1,6-Dichlorohexane | 1.5 | 91 |
| 19 | 2-Chlorohexane | 1.5 | 95 |
| 20 | 1,4-Dichlorobutene | 1.5 | 99 |
| 21 | Chlorocyclohexane | 3 | 3 |
| 22 | (3-Chlorobutyl)benzene | 1.5 | 91 |
| 23 | Benzyl chloride | 1.5 | 99 |
| 24 | Epichlorohydrin | 1.5 | 98 |
| 25 | Epichlorohydrin carbonate | 1.5 | 98 |

| | | | |
|---|---|---|---|
| Explanations to Table 1: ^{a} - amount of chlorine donor in equivalents, based on the amount of substrate, and calculated based on the content of chlorine; the chlorine content of the post-consumer waste was determined by Schöniger combustion as described above; ^{b} - yield in % of 10-chlorobenzoquinoline, determined by GC. | | | |

According to the general reaction protocol described above, several syntheses as compiled in Table 2 were performed using PVC water pipe as chlorine donor and different substrate compounds as shown in Table 3, and the yields as shown in Table 2 of the products shown in Table 4 were obtained in these syntheses.

**Table 2: Syntheses performed using different substrates and respective yields**

| Exp. No. | Substrate^{a} | Products^{b} | | Yield^{c} | |
|---|---|---|---|---|---|
| | | Dichlorinated | Monochlorinated | Dichlorinated | Monochlorinated |
| 26 | 1a | 1b | 1b' | 0 | 99 |
| 27 | 2a | 2b | 2b' | 95 | 3 |
| 28 | 3a | 3b | 3b' | 90 | 8 |
| 29 | 4a | 4b | 4b' | 96 | 1 |
| 30 | 5a | 5b | 5b' | 73 | 14 |
| 31 | 6a | 6b | 6b' | 74 | 22 |
| 32 | 7a | 7b | 7b' | 90 | 4 |
| 33 | 8a | 8b | 8b' | 0 | 98 |
| 34 | 9a | 9b | 9b' | 0 | 68 |
| 35 | 10a | 10b | 10b' | 2 | 95 |
| 36 | 11a | 11b | 11b' | 89 | 10 |
| 37 | 12a | 12b | 12b' | 84 | 15 |
| 38 | 13a | 13b | 13b' | 11 | 85 |
| 39 | 14a | 14b | 14b' | 0 | 97 |
| 40 | 15a | 15b | 15b' | 0 | 92 |
| 41 | 16a | 16b | 16b' | 45 | 52 |
| 42 | 17a | 17b | 17b' | 41 | 56 |
| 43 | 18a | 18b | 18b' | 18 | 72 |
| 44 | 19a | 19b | 19b' | 9 | 81 |
| 45 | 20a | 20b | 20b' | 20 | 76 |
| 46 | 21a | 21b | 21b' | 20 | 72 |
| 47 | 22a | 22b | 22b' | 16 | 81 |
| 48 | 23a | 23b | 23b' | 10 | 76 |
| 49 | 24a | 24b | 24b' | 0 | 75 |
| 50 | 25a | 25b | 25b' | 0 | 78 |
| 51 | 26a | 26b | 26b' | 0 | 60 |
| 52 | 27a | 27b | 27b' | 0 | 60 |

| | | | | | |
|---|---|---|---|---|---|
| Explanations to Table 2: ^{a} - see Table 3 for structures; ^{b} - see Table 4 for structures; ^{c} -product yield in % as determined by GC. | | | | | |

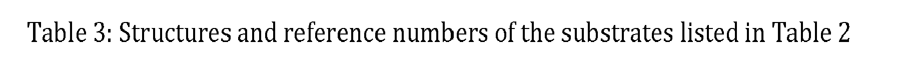

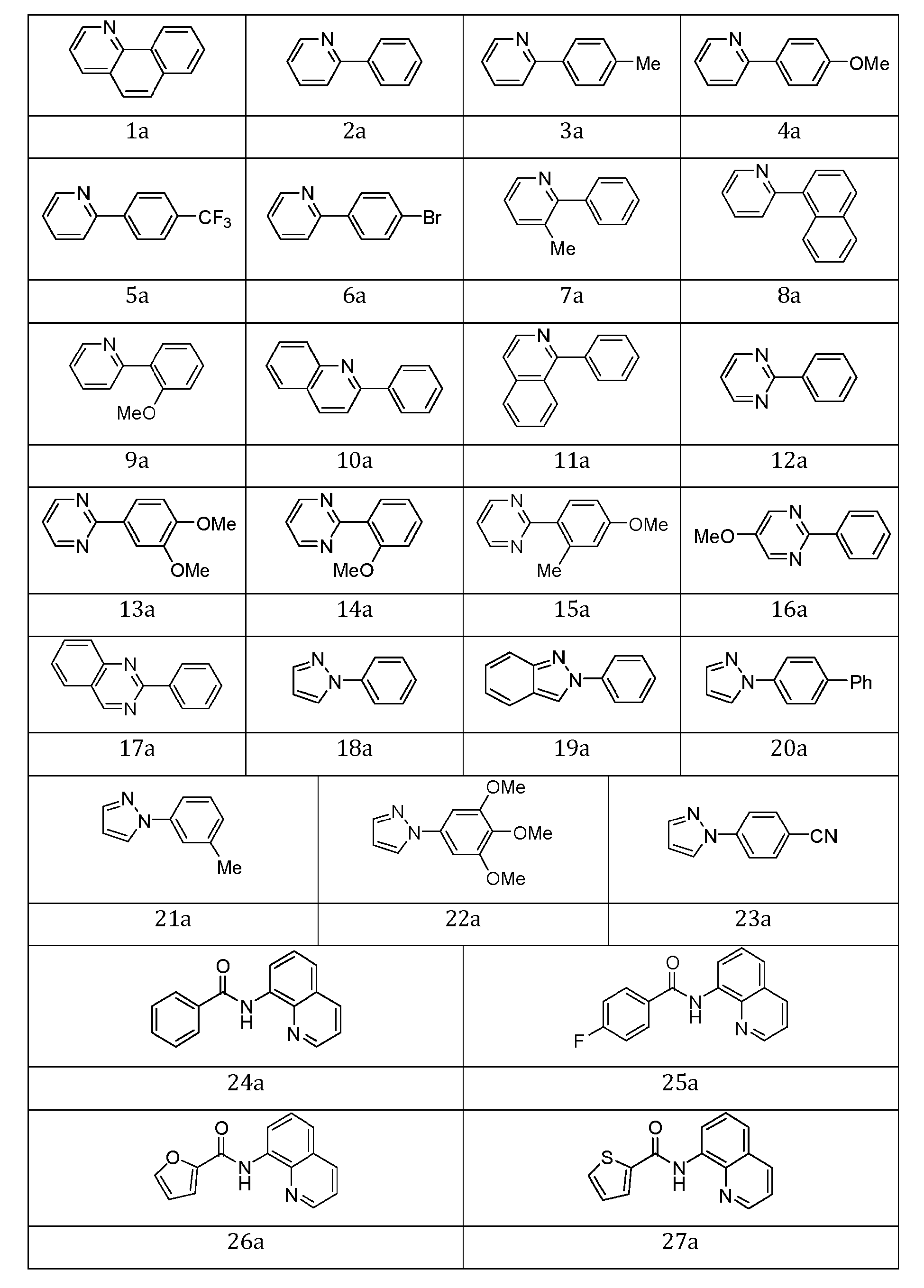

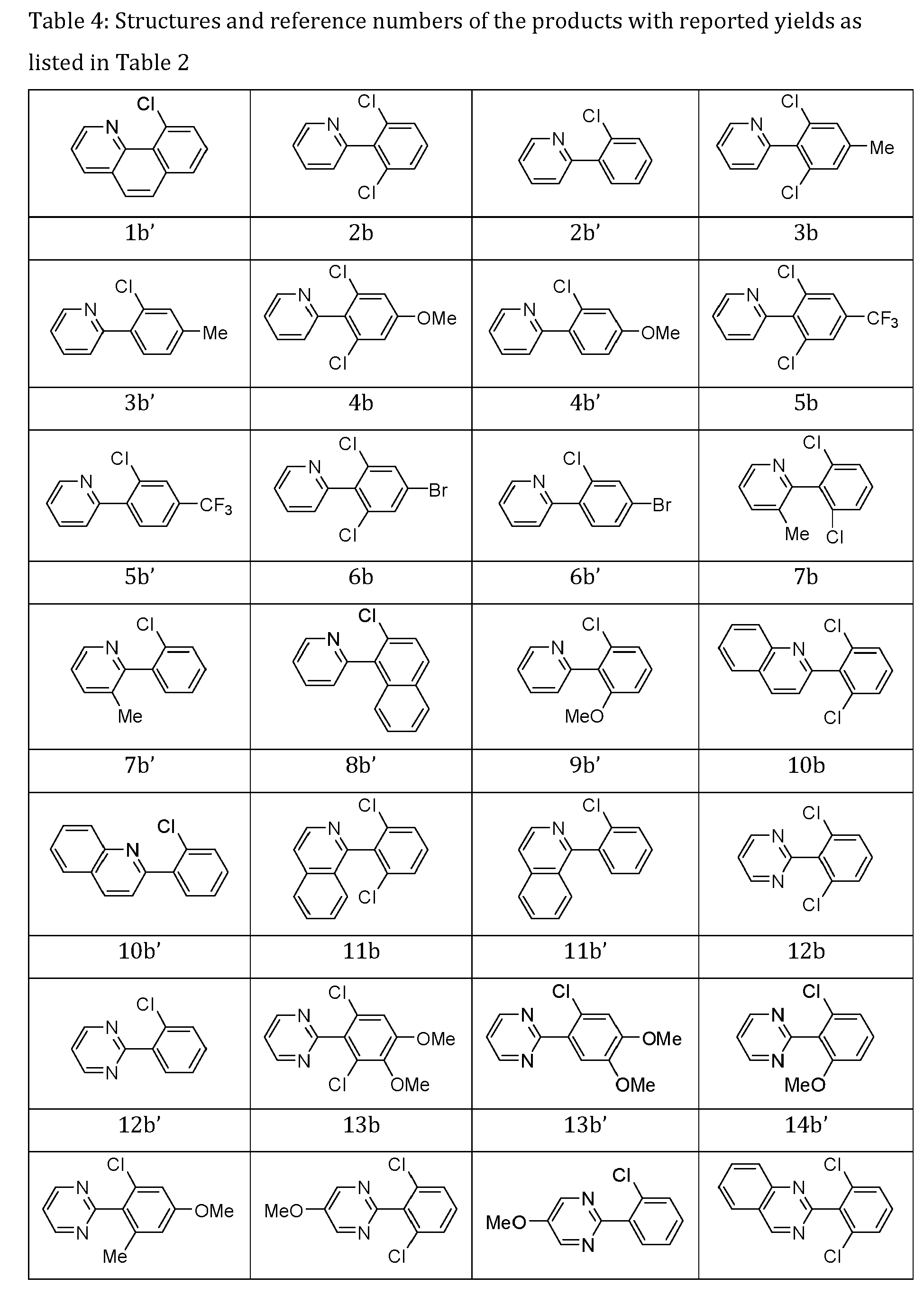

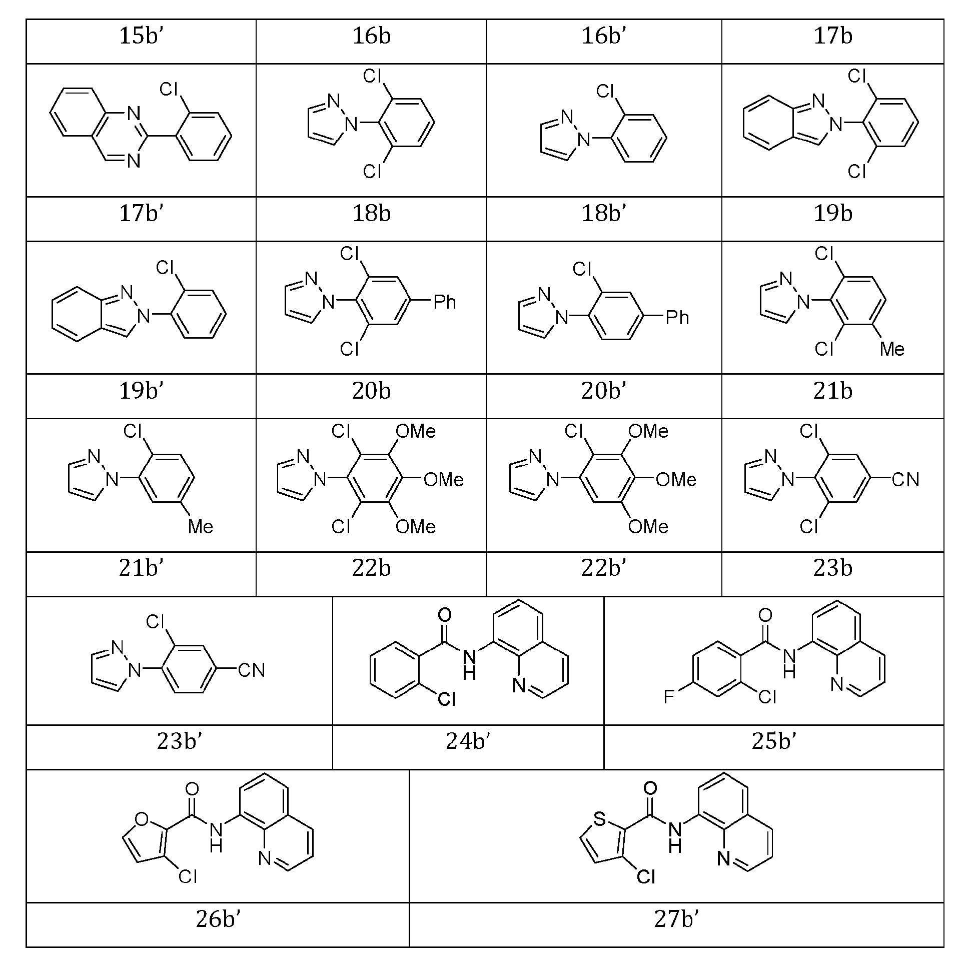

The ¹H and ¹³C NMR peaks of the products are listed below.
10-chlorobenzoquinoline (1b'):
   ¹H NMR (400 MHz, DMSO) δ=9.07 (dd, *J*=4.3, 1.9 Hz, 1H), 8.46 (dd, *J*=8.0, 1.9 Hz, 1H), 8.07 (dd, *J*=7.9, 1.4 Hz, 1H), 8.01 (d, *J*=8.8 Hz, 1H), 7.95 (d, *J*=8.8 Hz, 1H), 7.87 (dd, *J*=7.7, 1.3 Hz, 1H), 7.77-7.66 ppm (m, 2H). ¹³C NMR (101 MHz, DMSO) δ=148.31, 145.85, 136.59, 136.53, 131.83, 131.51, 128.64, 128.50, 127.76, 127.48, 126.97, 122.76 ppm.
2-(2,6-dichlorophenyl)pyridine (2b):
   ¹H NMR (400 MHz, DMSO) δ=8.73 - 8.68 (m, 1H), 7.94 (t, *J* = 7.7 Hz, 1H), 7.59 (d, *J* = 8.1 Hz, 2H), 7.46 (dt, *J* = 17.0, 8.3 Hz, 3H). ¹³C NMR (101 MHz, DMSO) δ=155.29, 149.96, 138.66, 137.32, 134.07, 131.23, 128.82, 125.31, 123.86.
2-(2,6-dichloro-4-methylphenyl)pyridine (3b):
   ¹H NMR (400 MHz, DMSO) δ=8.70 (m, *J*=4.9, 1.8, 1.0 Hz, 1H), 7.91 (td, *J*=7.7, 1.8 Hz, 1H), 7.47-7.34 (m, 4H), 2.35 ppm (d, *J*=0.9 Hz, 3H).¹³C NMR (101 MHz, DMSO) δ 155.35, 149.91, 141.48, 137.15, 135.81, 133.65, 129.13, 125.45, 123.67, 20.65 ppm.
2-(2,6-dichloro-4-methoxyphenyl)pyridine (4b):
   ¹H NMR (400 MHz, DMSO) δ=8.68 (dt, *J*=4.8, 1.4 Hz, 1H), 7.91 (td, *J*=7.7, 1.9 Hz, 1H), 7.47-7.35 (m, 2H), 7.19 (s, 2H), 3.85 ppm (s, 3H).¹³C NMR (101 MHz, DMSO) δ=160.08, 155.22, 149.89, 137.19, 134.56, 131.19, 125.84, 123.65, 114.63, 56.62 ppm.
2-(2,6-dichloro-4-(trifluoromethyll)phenyl)pyridine (5b):
   ¹H NMR (400 MHz, DMSO) δ=8.74 (m, *J*=4.8, 1.8, 1.1 Hz, 1H), 8.03 (d, *J*=0.8 Hz, 2H), 7.98 (td, *J*=7.7, 1.8 Hz, 1H), 7.49 ppm (m, *J*=7.7, 5.6, 1.1 Hz, 2H).¹³C NMR (101 MHz, DMSO) δ=154.24, 150.17, 142.50, 137.53, 135.34, 132.08, 131.74, 131.41, 131.08, 127.06, 125.82, 125.78, 125.75, 125.71, 125.03, 124.34, 124.27, 121.63, 118.91 ppm.
2-(2,6-dichloro-4-bromophenyl)pyridine (6b):
   ¹H NMR (400 MHz, DMSO) δ=8.71 (m, *J*=4.9, 1.9, 1.0 Hz, 1H), 7.95 (td, *J*=7.7, 1.8 Hz, 1H), 7.91 (s, 2H), 7.51-7.40 ppm (m, 2H).¹³C NMR (101 MHz, DMSO) δ=154.48, 150.10, 138.03, 137.45, 135.09, 131.27, 125.32, 124.09, 122.46 ppm.
2-(2,6-dichloro-phenyl)-3-methyl-pyridine (7b):
   ¹H NMR (400 MHz, DMSO) δ=8.52 (m, *J*=4.8, 1.6, 0.7 Hz, 1H), 7.80 (m, *J*=7.7, 1.7, 0.9 Hz, 1H), 7.62 (dd, *J*=8.0, 0.8 Hz, 2H), 7.50 (dd, *J*=8.8, 7.3 Hz, 1H), 7.39 (dd, *J*=7.8, 4.8 Hz, 1H), 2.03 ppm (s, 3H). ¹³C NMR (101 MHz, DMSO) δ=154.91, 147.55, 138.41, 137.99, 133.91, 132.09, 131.24, 128.81, 124.12, 18.05 ppm.
2-(2-chloronaphthalen-1-yl)pyridine (8b'):
   ¹H NMR (400 MHz, CDCl₃) δ=8.76 (m, *J*=4.9, 1.8, 1.0 Hz, 2H), 7.79 (m, *J*=8.5, 6.9, 4.9 Hz, 6H), 7.48 (d, *J*=8.8 Hz, 2H), 7.45-7.24 ppm (m, 10H).¹³C NMR (101 MHz, CDCl₃) δ=156.66, 149.83, 136.41, 136.10, 133.24, 132.12, 130.70, 129.76, 128.04, 127.18, 126.01, 125.69, 122.67 ppm,
2-(2-chloro-6-methoxyphenyl)pyridine (9b'):
   ¹H NMR (400 MHz, CDCl₃) δ=8.65 (m, *J*=4.9, 1.8, 1.0 Hz, 1H), 7.67 (td, *J*=7.7, 1.8 Hz, 1H), 7.27-7.16 (m, 3H), 7.01 (dd, *J*=8.1, 1.0 Hz, 1H), 6.81 (dd, *J*=8.4, 1.0 Hz, 1H), 3.64 ppm (s, 3H). ¹³C NMR (101 MHz, CDCl₃) δ=158.25, 154.97, 149.47, 136.05, 134.13, 129.79, 129.20, 125.67, 122.36, 121.82, 109.53, 77.31, 56.11 ppm.
3-(2-chlorophenyl)isoquinoline (10b'):
   ¹H NMR (400 MHz, CDCl₃) δ=8.19-8.07 (m, 2H), 7.81 (dd, *J*=8.0, 1.5 Hz, 1H), 7.72-7.64 (m, 2H), 7.66-7.58 (m, 1H), 7.51 (m, *J*=8.2, 6.9, 1.2 Hz, 1H), 7.44 (dd, *J*=7.2, 2.0 Hz, 1H), 7.33 ppm (pd, *J*=7.4, 1.8 Hz, 2H).¹³C NMR (101 MHz, CDCl₃) δ=157.45, 148.11, 139.70, 135.67, 132.39, 131.71, 130.11, 129.89, 129.72, 129.69, 127.57, 127.19, 127.15, 126.79, 122.79, 77.23 ppm.
1-(2,6-dichlorophenyl)isoquinoline (11b):
   ¹H NMR (400 MHz, CDCl₃) δ=8.61 (d, *J*=5.7 Hz, 1H), 7.86 (dt, *J*=8.3, 1.1 Hz, 1H), 7.72-7.59 (m, 2H), 7.51-7.38 (m, 4H), 7.32 ppm (dd, *J*=8.9, 7.2 Hz, 1H). ¹³C NMR (101 MHz, CDCl₃) δ=156.70, 142.49, 136.96, 136.36, 135.20, 130.45, 130.24, 128.18, 127.80, 127.16, 126.04, 121.01, 77.23 ppm.
2-(2,6-dichlorophenyl)pyrimidine (12b):

   ¹H NMR (400 MHz, DMSO) δ=9.01 (d, *J*=5.0 Hz, 2H), 7.66-7.59 (m, 3H), 7.54 ppm (dd, *J*=9.0, 7.1 Hz, 1H). ¹³C NMR (101 MHz, DMSO) δ=163.64, 158.35, 137.82, 133.32, 131.65, 128.78, 121.38 ppm.
2-(2-chloro-4,5-dimethoxyphenyl)pyrimidine (13b'):
   ¹H NMR (400 MHz, CDCl₃) δ=8.89 (d, *J*=4.9 Hz, 2H), 7.39 (s, 1H), 7.28 (t, *J*=4.9 Hz, 1H), 7.01 (s, 1H), 3.96 ppm (d, *J*=1.8 Hz, 6H). ¹³C NMR (101 MHz, CDCl₃) δ=165.34, 157.23, 157.01, 150.39, 147.83, 129.50, 124.69, 118.95, 114.06, 113.52, 77.23, 56.28, 56.18 ppm.
2-(2-chloro-6-methoxyphenyl)pyrimidine (14b'):
   ¹H NMR (400 MHz, CDCl₃) δ=8.83 (d, *J*=4.9 Hz, 2H), 7.30-7.17 (m, 2H), 7.02 (dd, *J*=8.1, 0.9 Hz, 1H), 6.84 (dd, *J*=8.4, 0.9 Hz, 1H), 3.68 ppm (s, 3H).¹³C NMR (101 MHz, CDCl₃) δ=164.23, 158.11, 157.31, 133.54, 130.30, 128.29, 121.77, 119.55, 109.60, 77.23, 56.16 ppm.
2-(2-chloro-4-methoxy-6-methylphenyl)pyrimidine (15b'):
   ¹H NMR (400 MHz, CDCl₃) δ=8.80 (d, *J*=4.9 Hz, 2H), 7.21 (t, *J*=4.9 Hz, 1H), 6.79 (d, *J*=2.5 Hz, 1H), 6.66 (dd, *J*=2.4, 0.9 Hz, 1H), 3.74 (s, 3H), 2.03 ppm (s, 3H). ¹³C NMR (101 MHz, CDCl₃) δ=166.22, 159.75, 157.24, 139.05, 133.27, 131.14, 119.27, 114.77, 112.23, 55.52, 20.34 ppm.
2-(2,6-dichlorophenyl)-5-methoxypyrimidine (16b):
   ¹H NMR (500 MHz, CDCl₃) δ=8.58 (s, 2H), 7.42 (d, *J*=8.0 Hz, 2H), 7.30 (dd, *J*=14.8, 6.5 Hz, 1H), 4.02 ppm (s, 3H). ¹³C NMR (126 MHz, CDCl₃) δ=156.51, 152.19, 143.52, 137.29, 134.49, 130.12, 128.08, 56.03 ppm.
2-(2-chlorophenyl)-5-methoxypyrimidine (16b'):
   ¹H NMR (500 MHz, CDCl₃) δ=8.56 (s, 3H), 7.72 (dd, *J*=6.1, 3.4 Hz, 2H), 7.55-7.48 (m, 2H), 7.45-7.28 (m, 4H), 4.01 ppm (s, 5H).¹³C NMR (126 MHz, CDCl₃) δ=158.22, 151.90, 143.53, 143.20, 137.44, 132.63, 131.55, 130.51, 130.03, 128.08, 126.79, 56.03 ppm.
2-(2,6-dichlorophenyl)quinazoline (17b):
   ¹H NMR (500 MHz, CDCl₃) δ=9.59 (s, 1H), 8.18 (d, *J*=8.5 Hz, 1H), 8.09-7.99 (m, 2H), 7.78 (m, *J*=8.1, 6.9, 1.1 Hz, 1H), 7.47 (d, *J*=8.1 Hz, 2H), 7.36 ppm (dd, *J*=8.7, 7.5 Hz, 1H). ¹³C NMR (126 MHz, CDCl₃) δ=160.86, 160.38, 150.35, 137.90, 134.64, 134.26, 130.25, 128.71, 128.53, 128.19, 127.30, 123.61 ppm.
2-(2-chlorophenyl)quinazoline (17b'):
   ¹H NMR (500 MHz, CDCl₃) δ=9.58 (d, *J*=16.0 Hz, 1H), 8.16 (d, *J*=8.2 Hz, 1H), 8.09-7.96 (m, 3H), 7.89-7.82 (m, 1H), 7.82-7.70 (m, 1H), 7.60-7.53 (m, 1H), 7.50-7.33 ppm (m, 3H).¹³C NMR (126 MHz, CDCl₃) δ=162.02, 160.29, 150.38, 138.30, 134.64, 134.44, 132.92, 131.81, 130.58, 130.36, 130.25, 128.71, 128.68, 128.54, 128.19, 128.11, 127.31, 127.19, 126.93, 123.31 ppm.
1-(2-chlorophenyl)-1H-pyrazole (18b'):

   ¹H NMR (400 MHz, CDCl₃) δ=7.81 (d, *J*=2.4 Hz, 3H), 7.68 (d, *J*=1.9 Hz, 3H), 7.56-7.47 (m, 3H), 7.47-7.35 (m, 3H), 7.35-7.21 (m, 6H), 6.47-6.37 ppm (m, 3H).¹³C NMR (101 MHz, CDCl₃) δ=140.93, 131.30, 130.66, 130.63, 129.00, 128.68, 128.37, 127.82, 127.67, 106.69, 106.66 ppm.
2-(2-chlorophenyl)-2H-indazole (19b'):
   ¹H NMR (400 MHz, CDCl₃) δ=8.27 (d, *J*=1.0 Hz, 1H), 7.76-7.58 (m, 3H), 7.57-7.46 (m, 1H), 7.44-7.24 (m, 3H), 7.07 ppm (m, *J*=8.5, 6.6, 0.9 Hz, 1H).¹³C NMR (101 MHz, CDCl₃) δ=149.43, 138.64, 130.70, 129.97, 129.00, 128.59, 127.71, 126.95, 125.23, 122.45, 122.02, 120.53, 117.95, 77.23 ppm.
1-(3-chloro-[1,1'-biphenyl]-4-yl)-1H-pyrazole (20b'):
   ¹H NMR (400 MHz, CDCl₃) δ=7.86 (dd, *J*=2.4, 0.6 Hz, 1H), 7.69 (dd, *J*=11.4, 1.9 Hz, 2H), 7.63-7.48 (m, 4H), 7.46-7.29 (m, 3H), 6.43 ppm (dd, *J*=2.5, 1.9 Hz, 1H). ¹³C NMR (101 MHz, CDCl₃) δ=142.22, 141.01, 138.79, 137.13, 131.32, 129.11, 129.06, 128.40, 128.27, 127.92, 127.12, 126.30, 106.75, 77.23 ppm.
1-(2-chloro-5-methylphenyl)-1H-pyrazole (21b'):
   ¹H NMR (400 MHz, CDCl₃) δ=7.90 (d, *J*=2.5 Hz, 1H), 7.76 (d, *J*=1.8 Hz, 1H), 7.46-7.38 (m, 2H), 7.16 (dd, *J*=8.4, 2.2 Hz, 1H), 6.49 (t, *J*=2.2 Hz, 1H), 2.40 ppm (s, 3H). ¹³C NMR (101 MHz, CDCl₃) δ=140.79, 138.01, 137.79, 131.29, 130.27, 129.72, 128.26, 124.97, 106.55, 77.23, 20.77 ppm.
1-(2-chloro-3,4,5-trimethoxyphenyl)-1H-pyrazole (22b'):
   ¹H NMR (400 MHz, CDCl₃) δ=7.89 (dd, *J*=2.5, 0.7 Hz, 1H), 7.73 (dd, *J*=1.7, 0.6 Hz, 1H), 6.93 (s, 2H), 6.48 (dd, *J*=2.5, 1.8 Hz, 1H), 3.95 (s, 7H), 3.89 ppm (s, 3H). ¹³C NMR (101 MHz, CDCl₃) δ=153.77, 140.93, 136.71, 136.40, 127.02, 107.54, 97.29, 77.24, 61.05, 56.34 ppm.
3-chloro-4-(1H-pyrazol-1-yl)benzonitrile (23b'):

   ¹H NMR (400 MHz, CDCl₃) δ=8.01 (dd, *J*=2.6, 0.6 Hz, 1H), 7.80-7.70 (m, 3H), 7.61 (dd, *J*=8.4, 1.8 Hz, 1H), 6.47 ppm (dd, *J*=2.6, 1.8 Hz, 1H). ¹³C NMR (101 MHz, CDCl₃) δ=142.15, 141.55, 134.60, 131.41, 131.24, 127.86, 127.55, 116.90, 112.18, 107.93, 77.23 ppm.
2-chloro-N-(quinolin-8-yl)benzamide (24b'):
   ¹H NMR (400 MHz, CDCl₃) δ=10.61 (s, 1H), 8.85-8.78 (m, 2H), 8.52 (dd, *J*=8.6, 1.7 Hz, 1H), 8.04-7.96 (m, 2H), 7.58 (d, *J*=8.4 Hz, 1H), 7.56-7.43 ppm (m, 4H). ¹³C NMR (101 MHz, CDCl₃) δ=165.44, 148.79, 139.34, 134.91, 133.90, 133.50, 132.03, 128.87, 127.34, 127.30, 126.04, 124.51, 122.44, 116.49, 77.24 ppm.
2-chloro-4-fluoro-N-(quinolin-8-yl)benzamide (25b'):
   ¹H NMR (500 MHz, CDCl₃) δ=10.38 (s, 1H), 9.31 (dt, *J*=8.9, 1.5 Hz, 1H), 9.07-9.01 (m, 1H), 8.98-8.89 (m, 1H), 8.64 (dd, *J*=8.8, 1.6 Hz, 1H), 8.61 (s, 0H), 7.80-7.74 (m, 1H), 7.30-7.17 ppm (m, 4H).¹³C NMR (126 MHz, CDCl₃) δ=163.43, 162.44, 149.29, 139.81, 139.59, 137.60, 133.64, 133.41, 127.51, 124.83, 121.75, 116.49, 116.29, 114.51, 77.25 ppm.
3-chloro-N-(quinolin-8-yl)furan-2-carboxamide (26b'):
   ¹H NMR (500 MHz, CDCl₃) δ=10.75 (s, 1H), 8.95 (dd, *J*=4.1, 1.6 Hz, 1H), 8.84 (d, *J*=8.3 Hz, 1H), 8.61 (dd, *J*=8.6, 1.8 Hz, 1H), 7.69-7.63 (m, 2H), 7.62 (dd, *J*=8.5, 4.2 Hz, 1H), 7.33 (d, *J*=3.5 Hz, 1H), 6.62 ppm (dd, *J*=3.5, 1.7 Hz, 1H). ¹³C NMR (126 MHz, CDCl₃) δ=156.35, 148.91, 148.17, 144.66, 139.21, 133.50, 133.43, 127.28, 126.04, 124.65, 122.47, 116.57, 115.40, 112.55 ppm.
3-chloro-N-(quinolin-8-yl)thiophene-2-carboxamide (27b'):
   ¹H NMR (400 MHz, CDCl₃) δ=11.29 (s, 1H), 8.85 (dd, *J*=4.2, 1.6 Hz, 1H), 8.77 (d, *J*=8.4 Hz, 1H), 8.53 (dd, *J*=8.6, 1.6 Hz, 1H), 7.61-7.46 (m, 3H), 7.02 ppm (d, *J*=5.2 Hz, 1H). ¹³C NMR (101 MHz, CDCl₃) δ=158.69, 149.02, 139.47, 133.96, 133.82, 133.37, 130.20, 129.86, 127.27, 126.04, 125.02, 124.24, 122.46, 117.25, 77.23 ppm.

Further experiments were performed according to the general reaction protocol described above with varying reaction conditions using 7,8-benzoquinoline as substrate and PVC water pipe as chlorine donor as compiled in Table 5.

**Table 5: Syntheses performed with varying reaction conditions**

| Exp. No. | Catalyst^{a} | Second catalyst^{b} | NaNO₃^{c} | Gaseous oxidising agent | Temp.^{d} | Yield^{e} |
|---|---|---|---|---|---|---|
| 53 | CuO, 20 mol% | PdO, 5 mol% | 20 | O₂, 3 bar | 140 | 58 |
| 54 | CuSO₄, 20 mol% | PdO, 5 mol% | 20 | O₂, 3 bar | 140 | 38 |
| 55 | Cu(OAc)₂, 20 mol% | PdO, 5 mol% | 20 | O₂, 3 bar | 140 | 28 |
| 56 | Cu(NO₃)₂, 20 mol% | PdO, 5 mol% | 20 | O₂, 3 bar | 140 | 99 |
| 57 | Cu(OTf)₂, 20 mol% | PdO, 5 mol% | 20 | O₂, 3 bar | 140 | 37 |
| 58 | Cu(acac)₂, 20 mol% | PdO, 5 mol% | 20 | O₂, 3 bar | 140 | 36 |
| 59 | CuPF₆(CH₃CN)₂, 20 mol% | PdO, 5 mol% | 20 | O₂, 3 bar | 140 | 37 |
| 60 | PdO, 10 mol% | - | 100 | O₂, 3 bar | 140 | 7 |
| 61 | Cu(NO₃)₂, 5 mol% | PdO, 10 mol% | 100 | O₂, 3 bar | 140 | 28 |
| 62 | Cu(NO₃)₂, 10 mol% | PdO, 10 mol% | 100 | O₂, 3 bar | 140 | 41 |
| 63 | Cu(NO₃)₂, 20 mol% | PdO, 10 mol% | 100 | O₂, 3 bar | 140 | 73 |
| 64 | Cu(NO₃)₂, 30 mol% | PdO, 10 mol% | 100 | O₂, 3 bar | 140 | 73 |
| 65 | Cu(NO₃)₂, 20 mol% | - | 100 | O₂, 3 bar | 140 | 29 |
| 66 | Cu(NO₃)₂, 20 mol% | 10% Pd/C, 10 mol% | 100 | O₂, 3 bar | 140 | 74 |
| 67 | Cu(NO₃)₂, 20 mol% | Pd(OH)₂/C, 10 mol% | 100 | O₂, 3 bar | 140 | 75 |
| 68 | Cu(NO₃)₂, 20 mol% | Pd(OAc)₂, 10 mol% | 100 | O₂, 3 bar | 140 | 34 |
| 69 | Cu(NO₃)₂, 20 mol% | Pd(TFA)₂, 10 mol% | 100 | O₂, 3 bar | 140 | 39 |
| 70 | Cu(NO₃)₂, 20 mol% | Pd(dba)₂, 10 mol% | 100 | O₂, 3 bar | 140 | 25 |
| 71 | Cu(NO₃)₂, 20 mol% | Pd(acac)₂, 10 mol% | 100 | O₂, 3 bar | 140 | 52 |
| 72 | Cu(NO₃)₂, 20 mol% | Pd(NO₃)₂·2H₂O, 10 mol% | 100 | O₂, 3 bar | 140 | 39 |
| 73 | Cu(NO₃)₂, 20 mol% | PdSO₄, 10 mol% | 100 | O₂, 3 bar | 140 | 24 |
| 74 | Cu(NO₃)₂, 20 mol% | NiO, 10 mol% | 100 | O₂, 3 bar | 140 | 26 |
| 75 | Cu(NO₃)₂, 20 mol% | PdO, 2.5 mol% | 100 | O₂, 3 bar | 140 | 69 |
| 76 | Cu(NO₃)₂, 20 mol% | PdO, 5 mol% | 0 | O₂, 3 bar | 140 | 52 |
| 77 | Cu(NO₃)₂, 20 mol% | PdO, 5 mol% | 10 | O₂, 3 bar | 140 | 87 |
| 78 | Cu(NO₃)₂, 20 mol% | PdO, 5 mol% | 40 | O₂, 3 bar | 140 | 98 |
| 79 | Cu(NO₃)₂, 20 mol% | PdO, 5 mol% | 60 | O₂, 3 bar | 140 | 93 |
| 80 | Cu(NO₃)₂, 20 mol% | PdO, 5 mol% | 100 | O₂, 3 bar | 140 | 84 |
| 81 | Cu(NO₃)₂, 20 mol% | PdO, 5 mol% | 160 | O₂, 3 bar | 140 | 82 |
| 82 | Cu(NO₃)₂, 20 mol% | PdO, 5 mol% | 240 | O₂, 3 bar | 140 | 74 |
| 83 | Cu(NO₃)₂, 20 mol% | PdO, 5 mol% | 20 | O₂, 3 bar | 120 | 5 |
| 84 | Cu(NO₃)₂, 20 mol% | PdO, 5 mol% | 20 | O₂, 3 bar | 130 | 37 |
| 85 | Cu(NO₃)₂, 20 mol% | PdO, 5 mol% | 20 | O₂, 3 bar | 150 | 99 |
| 86 | Cu(NO₃)₂, 20 mol% | PdO, 5 mol% | 20 | O₂, 1 bar | 140 | 80 |
| 87 | Cu(NO₃)₂, 20 mol% | PdO, 5 mol% | 20 | O₂, 5 bar | 140 | 99 |
| 88 | Cu(NO₃)₂, 20 mol% | PdO, 5 mol% | 20 | Air, 1 bar | 140 | 3 |
| 89 | Cu(NO₃)₂, 20 mol% | PdO, 5 mol% | 20 | Air, 10 bar | 140 | 38 |
| 90^{f} | Cu(NO₃)₂, 20 mol% | PdO, 5 mol% | 20 | N₂, 1 bar | 140 | 0 |

Explanations to Table 5: ^{a} -amount of catalyst in mol% based on 7,8-benzoquinoline; ^{b} - amount of second catalyst in mol% based on 7,8-benzoquinoline; ^{c} - amount of NaNO₃ in mol% based on 7,8-benzoquinoline; ^{d} - reaction temperature in °C; ^{e} - yield in % of 10-chlorobenzoquinoline, determined by GC, ^{f} - comparative example.

As elucidated in the given examples, the chlorination of substrate compounds containing an arene moiety proceeds in the presence of a chlorine-donor, a catalyst, an oxidising agent, and a solvent. Good yields can be obtained for different catalysts and catalyst combinations. The method allows for a great flexibility concerning the oxidising agent. Moreover, waste products containing chlorinated hydrocarbon compounds can be used directly as chlorine-donor in the method according to the invention.

## Claims

1. Method for chlorination of a substrate compound containing an arene moiety with 3 to 20 carbon atoms, wherein the substrate compound is reacted with an organic chlorine-donor in the presence of a catalyst, an oxidising agent, and a solvent.

2. Method according to claim 1, wherein the chlorine-donor contains a chlorinated hydrocarbon compound.

3. Method according to claims 1 and 2, wherein the chlorinated hydrocarbon compound can be solid or liquid at 25°C, preferably wherein the chlorinated hydrocarbon compound contains a chlorine atom connected to an aliphatic moiety.

4. Method according to any of the preceding claims, wherein the chlorine-donor is selected from the group consisting of polyvinyl chloride, polyvinylidene chloride, polyepichlorohydrin, neoprene, poly(epichlorohydrin-co-ethylene oxide), poly(epichlorohydrin-co-CO₂), dichloromethane, chloroform, 1,2-dichloroethane, 1,1-dichloroethane, 1,1,2-trichloroethane, 1,2-dichloroethene, perchloroethene, 1,6-dichlorohexane, 2-chlorohexane, 1,4-dichlorobutene, chlorocyclohexane, (3-chlorobutyl)benzene, benzyl chloride, epichlorohydrin, epichlorohydrin carbonate, and mixtures thereof, preferably selected from the group consisting of polyvinyl chloride, polyvinylidene chloride, polyepichlorohydrin, neoprene, poly(epichlorohydrin-co-ethylene oxide), poly(epichlorohydrin-co-CO₂), dichloromethane, 1,2-dichloroethane, 1,1-dichloroethane, 1,2-dichloroethene, 1,6-dichlorohexane, 2-chlorohexane, 1,4-dichlorobutene, (3-chlorobutyl)benzene, benzyl chloride, epichlorohydrin, epichlorohydrin carbonate, and mixtures thereof.

5. Method according to any of the preceding claims, wherein the catalyst contains a metal, preferably contains a metal selected from the group consisting of copper, nickel, cobalt, palladium, and mixtures thereof, more preferably contains copper and/or palladium, even more preferably contains a palladium compound and/or a copper salt selected from the group consisting of PdO, Cu(OAc)₂, Cu(OTf)₂, CuSO₄, Cu(acac)₂, CuPF₆(CH₃CN)₂, CuO, and/or Cu(NO₃)₂ and mixtures thereof, most preferably contains Cu(NO₃)₂, and/or wherein the catalyst is a heterogeneous or homogeneous catalyst, preferably a homogeneous catalyst.

6. Method according to any of the preceding claims, wherein the catalyst is preferably used in combination with a second catalyst, preferably a second metal catalyst selected from the group consisting of a palladium catalyst, a platinum catalyst, an iron catalyst, a nickel catalyst such as NiO, and mixtures thereof, more preferably a palladium catalyst, even more preferably PdO, 10%Pd/C, Pd(OH)₂/C, Pd(OAC)₂, Pd(TFA)₂, Pd(dba)₂, Pd(acac)₂, Pd(NO₃)₂·2H₂O, and/or PdSO₄ and mixtures thereof, most preferably PdO.

7. Method according to any of the preceding claims, wherein the oxidising agent contains a compound selected from the group consisting of oxygen, air, nitrate, ozone, hydrogen peroxide, inorganic peroxides, chlorine, nitric acid, permanganate, dichromate, chlorate, sulfuric acid, osmium tetroxide, sodium perborate, nitrogen oxide gases, sodium bismuthate, ceric ammonium nitrate, ceric sulfate, lead dioxide, and mixtures thereof.

8. Method according to any of the preceding claims, wherein the oxidising agent is a combination of a gaseous and a non-gaseous oxidising agent, more preferably a combination of oxygen and a nitrate salt, in particular sodium nitrate,
and/or
wherein the solvent is a proton-containing non-aromatic solvent, preferably a dipolar aprotic solvent, preferably dimethyl sulfoxide, and/or wherein the solvent contains water, preferably contains water in an amount of 0.001 to 1 wt.%, more preferably 0.05 to 1 wt.%, most preferably 0.05 to 0.1 wt.% based on the amount of solvent.

9. Method according to any of the preceding claims, wherein the arene moiety of the substrate compound contains 1 to 8, preferably 1 to 3, heteroatoms selected from the group containing nitrogen, oxygen, sulfur, and phosphorus, preferably nitrogen.

10. Method according to any of the preceding claims, wherein the chlorination takes place on the arene moiety.

11. Method according to any of the preceding claims, wherein the substrate compound is reacted with the chlorine-donor at a temperature from 10 to 200 °C, preferably from 100 to 180 °C, more preferably from 130 to 150 °C, and/or wherein the chlorine-donor is employed in an amount of 1.0 to 5.0 equivalents, more preferably 1.0 to 3.0 equivalents, even more preferably 1.0 to 2.0 equivalents, most preferably 1.5 equivalents, based on the amount of substrate compound, and/or wherein the catalyst is employed in an amount of 1 to 50 mol% based on the amount of substrate compound, more preferably 5 to 40 mol%, even more preferably 10 to 30 mol%, most preferably 15 to 25 mol%.

12. Method according to any of claims 6 to 11, wherein the second catalyst is employed in an amount of 0.01 to 50 mol% based on the amount of substrate compound, more preferably 0.1 to 30 mol%, even more preferably 1 to 15 mol%, most preferably 2.5 to 7.5 mol%.

13. Method according to any of claims 7 to 12, wherein the non-gaseous oxidising agent is employed in an amount of 1 to 100 mol% based on the amount of substrate compound, preferably 5 to 80 mol%, more preferably 10 to 50 mol%, most preferably 20 mol%, and/or wherein the gaseous oxidising agent is employed at a pressure of 1 to 100 bar, preferably at 1 to 50 bar, more preferably at 1 to 10 bar, most preferably at 3 bar.

14. Use of a chlorine-containing solid or liquid organic compound, in particular a chlorinated organic waste compound, as chlorine-donor for the chlorination of a substrate compound containing an arene moiety with 3 to 20 carbon atoms.

15. Use according to claim 14, wherein the chlorine-containing solid or liquid organic compound is selected from solid polymer waste such as post-consumer waste plastic and rubber, liquid laboratory waste such as chlorinated solvents, and mixtures thereof, and/or wherein the chlorination is conducted in the presence of a catalyst, an oxidising agent, and a solvent, in particular as detailed in any one of claims 1 to 13.
